# EUROPEAN PATENT APPLICATION

(11) **EP 3 178 555 A1**
(43) Date of publication of application: **14.06.2017**
(21) Application number: 15199098.3
(22) Date of filing: 10.12.2015
(51) Int. Cl.: B01J 23/656, B01J 35/10, B01J 37/02, C07C 29/60, C07C 31/20

(54) **PROCESS FOR THE MANUFACTURE OF PROPANEDIOL**

(71) Applicant: Solvay SA, 1120 Bruxelles (BE)
(72) Inventor: SU, Fangzheng, 201100 Minhang district Shanghai (CN)
(74) Representative: Vande Gucht, Anne

(57) **Abstract**

A process for manufacturing 1,3-propanediol by reacting glycerol with hydrogen in the presence of a supported catalyst, the supported catalyst comprising at least one first element selected from iridium, rhodium, palladium, platinum and any combination thereof, at least one second element selected from chromium, molybdenum, tungsten and any combination thereof, and rhenium, said elements and rhenium being supported on alumina.

## Description

The invention relates to a process for the manufacture of 1,3-propanediol, in particular for the manufacture of 1,3-propanediol by hydrogenation of glycerol.

Trimethylene glycol (1,3-propanediol) is mainly used as a building block in the production of polymers such as polytrimethylene terephthalate and it can be formulated into a variety of industrial products including composites, adhesives, laminates, coatings, moldings, aliphatic polyesters and copolyesters. It can also be used as a solvent, an ingredient for a food composition, antifreeze and a wood paint. It can also be used in applications such as cosmetics, personal care, cleaning, agricultural compositions, engine coolants, food and beverages, deicing fluids and heat transfer fluids.

Trimethylene glycol may be chemically synthesized by the hydration of acrolein, by the hydroformylation of ethylene oxide to afford 3-hydroxypropionaldehyde, which is hydrogenated to give 1,3-propanediol, by bioprocessing of glucose and glycerol by certain micro-organisms, or by catalytic hydrogenation of glycerol.

International application WO 2015/022267 in the name of Solvay S.A. discloses the hydrogenolysis of glycerol to 1,3-propanediol over alumina supported platinum tungsten catalysts. Although selectivity towards 1,3-propanediol are satisfactory, opportunities remain to further improve the catalyst activity keeping a satisfactory 1,3-propanediol selectivity. This and other problems are solved by the present invention described below.

In a first embodiment, the invention is related to process for manufacturing 1,3-propanediol by reacting glycerol with hydrogen in the presence of a supported catalyst, the supported catalyst comprising at least one first element selected from iridium, rhodium, palladium, platinum and any combination thereof, at least one second element selected from chromium, molybdenum, tungsten and any combination thereof, and rhenium, said elements and rhenium being supported on alumina.

One of the essential features of the present invention is that the catalyst comprises rhenium.

This allows to increase the catalyst activity while keeping a satisfactory 1,3-propanediol selectivity and consequently to improve the process volumetric 1,3-propanediol productivity.

In a second embodiment, the invention relates to a process for making a polyester comprising obtaining 1,3-propanediol according to the process of the first embodiment and further submitting said 1,3-propanediol to a reaction with a carboxylic acid and/or a carboxylic acid ester.

In a third embodiment, the invention relates to a process for making a polyester fiber comprising obtaining a polyester according to the process of the second embodiment and further converting said polyester in to a fiber.

In a fourth embodiment, the invention relates to 1,3-propanediol obtainable according to the process of the first embodiment.

In a fifth embodiment, the invention relates to the use of the 1,3-propanediol of the fourth embodiment in the manufacture of a polyester.

In a sixth embodiment, the invention relates to a polyester obtainable according to the process of the fifth embodiment.

In a seventh embodiment, the invention relates to the use of the polyester of the sixth embodiment in the manufacture of a polyester fiber.

In an eight embodiment, the invention relates to a process for preparing a supported catalyst comprising at least one first element selected from iridium, rhodium, palladium, platinum and any combination thereof, at least one second element selected from chromium, molybdenum, tungsten and any combination thereof, and rhenium, said elements and rhenium being supported on alumina comprising:
a) Providing an alumina support;
b) Contacting said support with an aqueous solution containing at least one precursor of the second element and at least one precursor of rhenium;
c) Removing the water of b) in order to obtain a first dried solid;
d) Treating the first dried solid of c) with a gas containing oxygen in order to obtain a first calcined solid;
e) Optionally repeating b) to d) where the support is the calcined solid of d);
f) Contacting the first treated solid of d) or e) with an aqueous solution of at least one precursor of the first element;
g) Removing the water of f) in order to obtain a second dried solid;
h) Treating the second dried solid of g) with a gas containing oxygen in order to obtain a second calcined solid;
i) Contacting the second calcined solid of h) with a gas containing hydrogen in order to obtain the supported catalyst.

The present invention therefore provides processes and products as described below.
Item 1. A process for manufacturing 1,3-propanediol by reacting glycerol with hydrogen in the presence of a supported catalyst, the supported catalyst comprising at least one first element selected from iridium, rhodium, palladium, platinum and any combination thereof, at least one second element selected from chromium, molybdenum, tungsten and any combination thereof, and rhenium, said elements and rhenium being supported on alumina.
Item 2. The process according to item 1, wherein the alumina in the supported catalyst is selected from gamma alumina, delta alumina, theta alumina, and any mixture thereof.
Item 3. The process according to item 2, wherein the alumina in the supported catalyst is gamma alumina.
Item 4. The process according to item 2, wherein the alumina in the supported catalyst is delta alumina.
Item 5. The process according to item 2, wherein the alumina in the supported catalyst is theta alumina.
Item 6. The process according to item 2, wherein the alumina in the supported catalyst is selected from delta alumina, theta alumina, and any mixture thereof.
Item 7. The process according to any one of items 1 to 6, wherein in the supported catalyst, at least part of the first element is present under metallic form.
Item 8. The process according to any one of items 1 to 7, wherein in the supported catalyst, at least part of the second element is present under oxide form.
Item 9. The process according to any one of items 1 to 8, wherein in the supported catalyst, at least part of rhenium is present under oxide form.
Item 10. The process according to any one of items 1 to 6, wherein in the supported catalyst at least part of the first element is present under metallic form, at least part of the second element is present under oxide form and at least part of rhenium is present under oxide form.
Item 11. The process according to any one of items 1 to 10, wherein in the supported catalyst the first element is platinum.
Item 12. The process according to any one of items 1 to 11, wherein in the supported catalyst the second element is tungsten.
Item 13. The process according to any one of items 1 to 10, wherein in the supported catalyst the first element is platinum and the second element is tungsten.
Item 14. The process according to any one of items 1 to 13, wherein the content of the first element with respect to the supported catalyst and expressed in weight percent of metal per weight of catalyst higher than or equal to 0.1 percent by weight and lower than or equal to 20 % by weight.
Item 15. The process according to item 14, wherein the content of the first element with respect to the supported catalyst and expressed in weight percent of metal per weight of catalyst higher than or equal to 4 percent by weight and lower than or equal to 6 % by weight.
Item 16. The process according to any one of items 1 to 15, wherein the content of the second element with respect to the supported catalyst and expressed in weight percent of metal trioxide (MO₃) per weight of catalyst is higher than or equal to 1 percent by weight and lower than or equal to 20 % by weight.
Item 17. The process according to item 16, wherein the content of the second element with respect to the supported catalyst and expressed in weight of metal trioxide (MO₃) per weight of catalyst is higher than 9 % by weight and lower than or equal to 11 % by weight.
Item 18. The process according to any one of items 1 to 17, wherein the content of rhenium with respect to the supported catalyst and expressed in weight percent of metal per weight of catalyst higher than or equal to 0.1 percent by weight and lower than or equal to 10 % by weight.
Item 19. The process according to item 18, wherein the content of rhenium with respect to the supported catalyst and expressed in weight percent of metal per weight of catalyst higher than or equal to 0.5 percent by weight and lower than or equal to 8 % by weight.
Item 20. The process according to item 19, wherein the content of rhenium with respect to the supported catalyst and expressed in weight percent of metal per weight of catalyst higher than or equal to 2 percent by weight and lower than or equal to 6 % by weight.
Item 21. The process according to any one of items 1 to 20, wherein the supported catalyst exhibits at least one of the following features:
   - a nitrogen BET specific area higher than or equal to 50 m²/g and lower than or equal to 400 m²/g;
   - a nitrogen BET total pore volume higher than or equal to 0.2 cm³/g and lower than or equal to 1.5 cm³/g;
   - a nitrogen BET average pore size higher than or equal to 2 nm and lower than or equal to 150 nm;
   - a TEM average particle size of the first compound lower than or equal to 15 nm.
Item 22. The process according to any one of items 1 to 21 carried out in the continuous mode.
Item 23. The process according to any one of items 1 to 22, wherein the reaction is carried out in a liquid medium.
Item 24. The process according to any one of items 1 to 22, wherein the reaction is carried out in at least one of the following conditions:
   - A temperature higher than or equal to 70 °C and lower than or equal to 300 °C;
   - A hydrogen partial pressure higher than or equal to 1 bar absolute and lower than or equal to 200 bar absolute;
   - In a liquid medium at a residence time of the liquid medium higher than or equal to 5 min and lower than or equal to 25 h, when the process is carried out continuously.
Item 25. The process according to any one of items 1 to 24 carried out in a slurry reactor.
Item 26. The process according to any one of items 1 to 24 carried out in a trickle-bed reactor.
Item 27. A process for making a polyester comprising obtaining 1,3-propanediol according to the process of any one of items 1 to 26, and further submitting said 1,3-propanediol to a reaction with a carboxylic acid and/or a carboxylic acid ester.
Item 28. A process for making a polyester fiber comprising obtaining a polyester according to the process of item 27, and further converting said polyester into a fiber.
Item 29. 1,3-propanediol obtainable by the process of any one of items 1 to 26.
Item 30. Use of the 1,3-propanediol according to item 29 in the manufacture of a polyester.
Item 31. A polyester obtainable by the process of item 30.
Item 32. Use of the polyester according to item 31 in the manufacture of a polyester fiber.
Item 33. A process for preparing a supported catalyst comprising at least one first element selected from iridium, rhodium, palladium, platinum and any combination thereof, at least one second element selected from chromium, molybdenum, tungsten and any combination thereof, and rhenium, said elements and rhenium being supported on alumina comprising:
   a) Providing an alumina support;
   b) Contacting said support with an aqueous solution containing at least one precursor of the second element and at least one precursor of rhenium;
   c) Removing the water of b) in order to obtain a first dried solid;
   d) Treating the first dried solid of c) with a gas containing oxygen in order to obtain a first calcined solid;
   e) Optionally repeating b) to d) where the support is the calcined solid of d);
   f) Contacting the first calcined solid of d) or e) with an aqueous solution of at least one precursor of the first element;
   g) Removing the water of f) in order to obtain a second dried solid;
   h) Treating the second dried solid of g) with a gas containing oxygen in order to obtain a second calcined solid;
   i) Contacting the second calcined solid of h) with a gas containing hydrogen in order to obtain the supported catalyst.

In the process according to the first embodiment of the invention, the reaction may be carried out in a liquid medium or a gaseous reaction medium, preferably in a liquid medium.

The liquid medium may be a single-phase or multi-phase medium.

The liquid medium is composed of all of the dissolved or dispersed solid compounds, dissolved or dispersed liquid compounds and dissolved or dispersed gaseous compounds at the temperature of the reaction.

The liquid medium comprises the reactants, the catalyst, the solvent if any, the impurities present in the reactants, in the solvent if any and in the catalyst, the reaction intermediates, the product and the by-products of the reaction.

The reactants are the glycerol and the hydrogen. The product is 1,3-propanediol. The catalyst, the reaction intermediates, the by-products and the solvent are as described here below.

In the process according to the first embodiment of the invention, when the reaction is carried out in a liquid medium, said liquid reaction medium preferably contains less than 900 g of water per kg of liquid medium. The content of water in the liquid medium is more preferably lower than or equal to 800 g/kg, yet more preferably lower than or equal to 600 g/kg, still more preferably lower than or equal to 400 g/kg, even more preferably lower than or equal to 200 g/kg and most preferably lower than or equal to 100 g/kg. That content of water is usually higher than or equal to 3 g/kg of liquid medium, preferably higher than or equal to 5 g/kg, more preferably higher than or equal to 8 g/kg, yet more preferably higher than or equal to 10 g/kg, still more preferably higher than or equal to 50 g/kg, most preferably higher than or equal to 75 g/kg, yet most preferably higher than or equal to 90g/kg and still most preferably higher than or equal to 95 g/kg. A water content higher than or equal to 200 g/kg and lower than or equal to 800 g/kg, preferably higher than or equal to 250 g/kg and lower than or equal to 600 g/kg, and more preferably higher than or equal to 300 g/kg and lower than or equal to 500 g/kg is also convenient.

Water can be water generated by the hydrogenation reaction or external water e.g. water present in the reactants, the catalyst, the solvent if any or added water.

The water content of the liquid medium can be measured by any method like for instance Karl Fischer titration.

The water content can also be calculated as the sum of the water fed to the liquid medium together with the reactants, e.g. with the glycerol, the catalyst and/or the solvent and the water generated by the hydrogenation reaction. The water generated by the hydrogenation reaction can be obtained from the conversion of glycerol and the selectivity of the various products formed. For instance, each mole of glycerol converted into propanediol will generate one mole of water, each mole of glycerol converted into propanol will generate two moles of water, *etc.*

In the process according to the first embodiment of the invention, the glycerol can be synthetic glycerol or natural glycerol or any mixture thereof. Synthetic glycerol is glycerol which has been obtained from non-renewable raw materials. The glycerol is preferably natural glycerol *i.e.* glycerol which has been prepared in a conversion process of renewable raw materials. By glycerol which has been prepared in a conversion process of renewable raw materials one intends to denote glycerol obtained in a process selected from the group consisting of hydrolysis, saponification, transesterification, aminolysis and hydrogenation of oils and/or fats of animal and/or plant and/or algae origin, fermentation, hydrogenation and hydrogenolysis of mono- and polysaccharides and derived alcohols, derived from or occurring naturally in the biomass, and any combination thereof Glycerol which has been obtained during the manufacture of biodiesel, *i.e*. during the transesterification of oils and/or fats of animal and/or plant, and preferably during the transesterification of oils and/or fats of plant origin, is particularly convenient. Glycerol which has been obtained in the manufacture of biodiesel is more particularly convenient.

In the process according to the first embodiment of the invention, the hydrogen can be obtained from any source. The hydrogen is preferably molecular hydrogen.

In the process according to the first embodiment of the invention, the hydrogen is preferably obtained from at least one process selected from the group consisting of steam reforming of hydrocarbons, partial oxidation of hydrocarbons, autothermal reforming of hydrocarbons, water-gas shift, coal gasification, pyrolysis of organic waste products (tar, lignite pitch, petroleum distillation residues, plastics, rubber, cellulose, paper, textile, wood, straw, mixed municipal waste...) and co-pyrolysis of organic wastes products with coal (including bituminous coal, lignite,...), thermal and non-thermal plasma cracking of mixtures of water or steam, and fuels, biomass gasification, biomass pyrolysis and subsequent gasification, thermal or catalytic decomposition of nitrogen compounds like ammonia, hydrazine, biochemical hydrogen fermentation, enzymatic treatment of natural sugars (e.g. xylose), steam reforming of alcohols, for instance monoalcohols, such methanol or ethanol, and polyols, such as propanediols and glycerine, alkaline cracking of insaturated fatty acid particularly oleic and ricinoleic acid, electrolysis of an aqueous solution of a hydrogen halide, electrolysis of an aqueous solution of a metal halide like for instance sodium chloride or potassium chloride, hydrolysis of metals or metal hydrides, water splitting from for instance alkaline electrolysis, proton-exchange membrane electrolysis, solid oxide electrolysis, high pressure electrolysis, high temperature electrolysis, photoelectrochemical water splitting, photocatalytic water splitting, photobiological water splitting, solar thermochemical hydrogen production and water thermolysis. When the selected process is electrolysis of an aqueous solution of a hydrogen halide, the hydrogen halide is often selected from hydrogen chloride, hydrogen fluoride and any mixture thereof, and is frequently hydrogen chloride. When the selected process is electrolysis of an aqueous solution of sodium chloride or potassium chloride, electrolysis can be any of mercury electrolysis, membrane electrolysis or diaphragm electrolysis. Membrane electrolysis is preferred.

In the process according to the first embodiment of the invention, the hydrogen can be used in admixture with another compound. The other compound is usually selected from the group consisting of nitrogen, helium, argon, carbon dioxide, steam, saturated hydrocarbons, and any mixture thereof. In the process according to the first embodiment of the invention, the mixture containing hydrogen comprises generally at least 10 % by volume of hydrogen, preferably at least 50 % by volume, more preferably at least 75 % by volume, yet more preferably at least 90 % by volume, still more preferably at least 95 % by volume, most preferably at least 99 % by volume and still most preferably at least 99.9 % by volume. That mixture comprises generally at most 99.99 % of hydrogen by volume. A mixture which consists essentially of hydrogen is also convenient. A mixture which consists of hydrogen is also suitable.

In the process according to the first embodiment of the invention, by alumina, one intends to denote an aluminum hydroxide, an aluminum oxide, an aluminum oxy-hydroxide, any compound resulting from a thermal treatment, especially from a hydrothermal treatment, of aluminum hydroxide, and any mixture thereof, as defined in Ullmann's Encyclopedia of Industrial Chemistry, http://onlinelibrary.wiley.com/doi/10.1002/14356007.a01_557/pdf L.K. Hudson et al., published on line 15 JUN 2000, pages 607-645. Aluminum trihydroxides, aluminum oxide hydroxides and pseudoboehmite are examples of an aluminum hydroxide. Gibbsite, Bayerite or Nordstrandite are examples of an aluminum trihydroxide. Boehmite or Diaspore are examples of an aluminum oxide hydroxide. Corundum is an example of an aluminum oxide. Chi-, kappa-, gamma-, delta-, theta-, and eta-alumina are examples of compound resulting from a thermal treatment, especially a hydrothermal treatment, of an aluminum hydroxide.

In the process according to the first embodiment of the invention, the alumina in the supported catalyst is preferably selected from chi-, kappa-, gamma-, delta-, theta-, eta-alumina, and any mixture thereof, more preferably from gamma-, delta-, theta-alumina, and any mixture thereof, yet more preferably from gamma alumina, delta and theta alumina, still more preferably from gamma alumina and most preferably from delta-, theta-alumina and any mixture thereof. An alumina in the supported catalyst containing less than 10 percent by weight of gamma-alumina is also convenient.

In the process according to the first embodiment of the invention, the alumina can be crystalline, amorphous or a mixture thereof, according to X-ray diffraction analysis. The alumina is preferably a mixture of crystalline and amorphous alumina. X-ray diffraction patterns of crystalline alumina can be found in http://www.sasoltechdata.com/tds/PURALOX_CATALOX.pdf.

In the process according to the first embodiment of the invention, the supported catalyst, preferably exhibits at least one of the following features:
- a nitrogen BET (Brunauer Emmet Teller) specific area higher than or equal to 50 m²/g and lower than or equal to 400 m²/g;
- a nitrogen BET total pore volume higher than or equal to 0.2 cm³/g and lower than or equal to 1.5 cm³/g;
- a nitrogen BET average pore size higher than or equal to 2 nm and lower than or equal to 100 nm;
- a TEM (Transmission Electron Spectroscopy) average particle size of the first compound lower than or equal to 15 nm;
- a content of the at least one first compound with respect to the catalyst higher than or equal to 0.1 percent by weight and lower than or equal to 20 % by weight; and
- a content of the at least one second compound expressed in weight of trioxide per weight of catalyst higher than or equal to 1 percent by weight and lower than 20 percent by weight.

In the process according to the first embodiment of the invention, the first element is preferably selected from palladium, platinum and combination thereof, and is most preferably platinum.

In the process according to the first embodiment of the invention, the content of the first element in the supported catalyst with respect to the catalyst is usually higher than or equal to 0.1 % by weight, preferably higher than or equal to 0.5 % by weight, more preferably higher than or equal to 1 % by weight, yet more preferably higher than or equal to 2 % by weight, and most preferably higher than or equal to 4% by weight. That content is usually lower than or equal to 20 % by weight, preferably lower than or equal to 15% by weight, more preferably lower than or equal to 10% by weight, yet more preferably lower than or equal to 8% by weight and most preferably lower than or equal to 6% by weight. When more than one first elements are present, the above mentioned content applies to the sum of the first elements. The weight percent are expressed in weight of metal *i.e*. iridium (Ir) and/or rhodium (Rh) and/or palladium (Pd) and/or platinum (Pt) per weight of catalyst. A catalyst with 2.5 % by weight of Rh and 2.5 % by weight of Pt is an example of a catalyst having a weight percent content of the first element of 5 %. A catalyst with 5 % by weight of Pt is another example of a catalyst having a weight percent content of the first element of 5 %. These features are especially suited when the first element is platinum. A catalyst with a content of about 5 weight percent of the first element, preferably platinum, is particularly convenient.

In the process according to the first embodiment of the invention, the second element is more preferably selected from molybdenum, tungsten, and combination thereof, and is most preferably tungsten.

In the process according to the first embodiment of the invention, the content of the second element in the supported catalyst with respect to the catalyst is usually higher than or equal to 1 % by weight, preferably higher than or equal to 2 % by weight, more preferably higher than or equal to 5 % by weight, yet more preferably higher than or equal to 8 % by weight, and most preferably higher than or equal to 9% by weight. That content is usually lower than 20% by weight, preferably lower than or equal to 17 % by weight, yet more preferably lower than or equal to 15% by weight and most preferably lower than or equal to 11% by weight. When more than one second elements are present, the above mentioned content applies to the sum of the second elements. The weight percent are expressed in weight of metal trioxide, *i.e*. chromium trioxide (CrO₃) and/or, molybdenum trioxide (MoO₃) and/or tungsten trioxide (WO₃) per weight of catalyst. A catalyst with 5 % by weight of Cr expressed as CrO₃ and 5 % by weight of W expressed WO₃ is an example of a catalyst having a weight percent content of the second element expressed as trioxide of 10 %. A catalyst with 10 % by weight of W expressed as WO₃ is another example of a catalyst having a weight percent content of the second element expressed as metal trioxide compound of 10 %. These features are especially suited when the second element is tungsten. A catalyst with a content of about 10 weight percent of the second element, preferably tungsten, is particularly convenient.

In the process according to the first embodiment of the invention, the content of rhenium in the supported catalyst with respect to the catalyst is usually higher than or equal to 0.5 % by weight, preferably higher than or equal to 0.8 % by weight, more preferably higher than or equal to 1 % by weight, yet more preferably higher than or equal to 2 % by weight, and most preferably higher than or equal to 4% by weight. That content is usually lower than 15% by weight, preferably lower than or equal to 10 % by weight, yet more preferably lower than or equal to 8% by weight and most preferably lower than or equal to 6% by weight. The weight percent is expressed in weight of metal *i.e.* rhenium (Re) per weight of catalyst. A catalyst with a content of about 4 weight percent of rhenium is particularly convenient.

In the process according to the first embodiment of the invention, at least one part of the first element in the supported catalyst is preferably present under the metallic form. The part present under metallic form with respect to the total amount of the first element expressed in weight per cent is preferably higher than or equal to 5 %, more preferably higher than or equal to 10 %, still more preferably higher than or equal to 50 %, yet preferably higher than or equal to 90 %, most preferably higher than or equal to 95 %, and yet most preferably higher than or equal to 99 %. The weight percent are expressed as above for the first element. A catalyst where the first element is essentially under metallic form is particularly convenient. These features are especially suited when the first element is platinum.

In the process according to the first embodiment of the invention, at least one part of the second element in the supported catalyst is preferably present as an oxide. The part present under oxide form with respect to the total amount of the second element expressed in weight percent is preferably higher than or equal to 5 %, more preferably higher than or equal to 10 %, still more preferably higher than or equal to 50 %, yet preferably higher than or equal to 90 %, most preferably higher than or equal to 95 %, and yet most preferably higher than or equal to 99 %. The weight percent are expressed as above for the second element. A catalyst where the second element is essentially under oxide form is particularly convenient. These features are especially suited when the second element is tungsten.

In the process according to the invention, at least one part of rhenium in the supported catalyst is preferably present as a low valence rhenium oxide (ReOx). By low valence oxide, it is meant an oxide wherein the rhenium is at an oxidation state of less than or equal to six but not zero. The part of rhenium, present under low valence oxide form with respect to the total amount of rhenium compound, expressed in weight percent is preferably higher than or equal to 5 %, more preferably higher than or equal to 10 %, still more preferably higher than or equal to 50 %, yet preferably higher than or equal to 75 %, most preferably higher than or equal to 80 %, and yet most preferably higher than or equal to 90 %. The weight percent are expressed as above for rhenium. A catalyst where rhenium is essentially under low valence oxide form is particularly convenient.

Said contents of the first element, preferably platinum, under metallic form, of the second element, preferably tungsten, under trioxide form and of rhenium, preferably under low valence oxide form, can be determined by X-ray diffraction analysis, Temperature Programmed Reduction and EXFAS as disclosed in Nagawa et al. Journal of Catalysis: 272, 2010, 191-194 and by X-ray Photoelectron Spectroscopy.

In the process according to the first embodiment of the invention, the first element is preferably platinum, more preferably platinum metal, the second element is preferably tungsten, more preferably tungsten trioxide and rhenium is preferably a low valence oxide of rhenium.

In the process according to the first embodiment of the invention, the supported catalyst preferably contains less than 10 weight percent of titanium expressed as titanium dioxide.

In the process according to the first embodiment of the invention, the catalyst is usually in a non-powder form selected from the group consisting of rings, beads, spheres, saddles, pellets, tablets, extrudates, granules, crushed, flakes, honeycombs, filaments, cylinders, polygons and any mixture thereof, or in powder form. The preferred form depends usually of the type of reactor used. A powder form is preferred when a slurry or fluidized-bed reactor is used for carrying out the reaction, while non-powder forms are preferred when a fixed bed reactor or a trickle bed reactor is used for carrying out the reaction.

In the process according to the first embodiment of the invention, the reaction may be carried out in the absence or in the presence of a solvent. The solvent may be selected from the group consisting of inorganic solvent, organic solvent, and combinations thereof. Examples of inorganic solvents are water, supercritical carbon dioxide, and inorganic ionic liquids. Examples of organic solvents are alcohols, ethers, saturated hydrocarbons, esters, perfluorinated hydrocarbons, nitriles, amides and any mixture thereof. Examples of alcohols are methanol, ethanol, 1-propanol, 2-propanol, ethylene glycol, 1,2-propanediol, 1,3-propanediol. Examples of ethers are diethylene glycol, dioxane, tetrahydrofuran, ethylene glycol monomethyl ether, ethylene glycol monoethyl ether, ethylene glycol dimethyl ether, diethylene glycol monomethyl ether and diethylene glycol dimethyl ether. An example of saturated hydrocarbons is cyclohexane. An example of esters is ethyl acetate. Examples of perfluorinated hydrocarbons are perfluorinated alkane such as perfluorinated hexane, heptane, octane, nonane, cyclohexane, methylcyclohexane, dimethylcyclohexane or trimethylcyclohexane, perfluoroethers like Galden® HT from Solvay Solexis, perfluorotetrahydrofurane or perfluoroamine like FluorinertTM FC from 3M. An example of nitriles is acetonitrile. An example of amides is dimethylformamide.

In the process according to the first embodiment of the invention, when the reaction is carried out in the presence of a solvent, different from water, the content of the solvent in the liquid medium is usually higher than or equal to 1 g/kg of liquid reaction medium, preferably higher than or equal to 2 g/kg, more preferably higher than or equal to 5 g/kg, yet more preferably higher than or equal to 10 g/kg, still more preferably higher than or equal to 50 g/kg, most preferably higher than or equal to 100 g/kg, yet most preferably higher than or equal to 150g/kg and still most preferably higher than or equal to 200g/kg. That content of solvent is usually lower than or equal to 999 g/kg, preferably lower than or equal to 950 g/kg, more preferably lower than or equal to 900 g/kg, yet more preferably lower than or equal to 850 g/kg, still more preferably lower than or equal to 825g/kg and most preferably lower than or equal to 800 g/kg.

In the process according to the first embodiment of the invention, the reaction may also be carried out solvent-less, *i.e.* for a content of a solvent, different from water, from the product of reaction and from the by-products of reaction , in the liquid medium lower than 1 g/kg.

The process according to the first embodiment of the invention can be carried out according to any mode of operation. The mode of operation can be continuous or discontinuous. By continuous mode of operation, one intends to denote a mode of operation where the glycerol and hydrogen are added continuously in the process, and the 1,3-propanediol is continuously withdrawn from the process. Any other mode of operation is considered as discontinuous.

In the process according to the first embodiment of the invention, the reaction is preferably carried out in a liquid medium.

The process according to the first embodiment of the invention can be carried out in reaction apparatuses made of or coated with materials which are suitable for hydrogenation under pressure, resistant in the presence of corrosive compounds under the reaction conditions. Suitable materials can be selected from the group consisting of glass, enamel, enameled steel, graphite, impregnated graphite, like for example graphite impregnated with a perfluorinated polymer such as polytetrafluoroethylene, or graphite impregnated with a phenolic resin, polyolefins, like for instance polyethylene or polypropylene, fluorinated polymers, like perfluorinated polymers such as for instance polytetrafluoroethylene, poly(perfluoropropylvinylether), copolymers of tetrafluoroethylene and hexafluoropropylene, and like partially fluorinated polymers such as for instance poly(vinylidene fluoride), polymers comprising sulphur, like for instance polysulphones or polysulphides, metals, like for instance tantalum, titanium, copper, gold, silver, nickel and molybdenum, and metal alloys, like for instance serie 300 stainless steel such as stainless steel type 302, stainless steel type 304, stainless steel type 316, alloys containing nickel, such as Hastelloy B, Hastelloy C, alloys containing molybdenum, such as Inconel 600, Inconel 625 or Incoloy 825.

The materials may be used within the mass, or in the form of cladding, or else by means of any coating process. Enameled steel is particularly convenient. Glass-lined apparatuses are also convenient. Stainless steel type 316 and Hastelloy C and tantalum coated apparatuses are also convenient.

In the process according to the first embodiment of the invention, the reaction is carried out at a temperature preferably higher than or equal to 70°C, more preferably higher than or equal to 80°C, yet more preferably higher than or equal to 90°C and most preferably higher than or equal to 100°C. That temperature is preferably lower than or equal to 300°C, more preferably lower than or equal to 200°C, yet more preferably lower than or equal to 180°C and most preferably lower than or equal to 150°C. A temperature between 150 °C and 180°C is also particularly convenient.

In the process according to the first embodiment of the invention, the reaction is preferably carried out at a hydrogen partial pressure preferably higher than or equal to 1 bar absolute (1 bara), more preferably higher than or equal to 5 bara and yet more preferably higher than or equal to 10 bara, still more preferably higher than or equal to 20 bara, most preferably higher than or equal to 50 bara, and yet most preferably higher than or equal to 80 bara. That hydrogen partial pressure is preferably lower than equal to 200 bara, more preferably lower than equal to 150 bara and most preferably lower than equal to 120 bara. A pressure between 50 bara and 70 bara is also particularly convenient.

In the process according to the first embodiment of the invention carried out under continuous mode, the residence time which is the ratio of the volume of the liquid medium to the flow rate by volume of the liquid reactants, depends on the reaction rate, on the hydrogen partial pressure, on the temperature, on the thoroughness with which the liquid medium is mixed, and on the activity and concentration of the supported catalyst. This residence time is usually higher than or equal to 5 minutes, often higher than or equal to 15 minutes, frequently higher than or equal to 30 minutes and in particular higher than or equal to 60 minutes. This residence time is usually lower than or equal to 25 hours, often lower than or equal to 20 hours, frequently lower than or equal to 10 and in particular lower than or equal to 5 hours.

In the process according to the first embodiment of the invention carried out under continuous mode, especially when a gas and a liquid phase are present, the residence time for the liquid phase, which is the ratio of the volume of the reactor volume to the flow rate by volume of the liquid phase, is usually higher than or equal to 5 minutes, often higher than or equal to 15 minutes, frequently higher than or equal to 30 minutes and in particular higher than or equal to 60 minutes. This residence time is usually lower than or equal to 25 hours, often lower than or equal to 10 hours and frequently lower than or equal to 5 hours.

In the process according to the first embodiment of the invention carried out under continuous mode, especially when a gas and a liquid phase are present, the residence time for the gas phase, which is the ratio of the volume of the reactor volume to the flow rate by volume of the gas phase, is usually higher than or equal to 1 second, often higher than or equal to 7seconds, and in particular higher than or equal to 10seconds. This residence time is usually lower than or equal to 10 minutes, often lower than or equal to 5 minutes and frequently lower than or equal to 2 minutes.

In a Continuous Stirred-Tank Reactor or in a bubble column, the volume of the reactor in the above definition of the residence time of the liquid or the gas phase can be replaced by the volume of the reactor occupied by the liquid.

In a trickle-bed reactor, the volume of the reactor in the above definition of the residence time of the liquid or the gas phase can be replaced by the volume of the reactor occupied by the catalyst.

In the process according to the first embodiment of the invention carried out under discontinuous mode, the reaction time required for the process according to the invention depends on the reaction rate, on the hydrogen partial pressure, on the glycerol concentration, on the temperature, on the thoroughness with which the reaction mixture is mixed, and on the activity and concentration of the supported catalyst. The required reaction time is usually higher than or equal to 5 minutes, often higher than or equal to 15 minutes, frequently higher than or equal to 30 minutes, in particular higher than or equal to 60 minutes, and more specifically higher than or equal to 160 min. This reaction time is usually lower than or equal to 25 hours, often lower than or equal to 20 hours, frequently lower than or equal to 10 and in particular lower than or equal to 6 hours.

In the process according to the first embodiment of the invention carried out under discontinuous mode, especially when a gas and a liquid phase are present, the reaction time for the liquid phase is usually higher than or equal to 5 minutes, often higher than or equal to 15 minutes, frequently higher than or equal to 30 minutes, in particular higher than or equal to 60 minutes, and more specifically higher than or equal to 180 min. This reaction time is usually lower than or equal to 25 hours, often lower than or equal to 10 hours and frequently lower than or equal to 6 hours.

In the process according to the first embodiment of the invention carried out under discontinuous mode, especially when a gas and a liquid phase are present, the reaction time can be adjusted depending on the conversion sought for the glycerol and the selectivity sought for the 1,3-propanediol, under the reaction conditions, and that reaction time can vary between 6and 80 h.

In the process according to the first embodiment of the invention, especially when the reaction is carried out continuously, the molar ratio between the flow rates of hydrogen and glycerol is usually higher than or equal to 0.1 mol/mol, often higher than or equal to 0.5 mol/mol, frequently higher than or equal to 0.7 mol/mol and in particular higher than 1 mol/mol. This ratio is usually lower than or equal to 100 mol/mol, often lower than or equal to 50 mol/mol, frequently lower than or equal to 20 mol/mol and in particular lower than or equal to 10 mol/mol.

The process according to the first embodiment of the invention may be carried out in any type of reactor. The reactor, especially when the reaction is carried out in the presence of a catalyst, may be selected from the group consisting of a slurry reactor, a fixed bed reactor, a trickle bed reactor, a fluidized bed reactor, and combinations thereof. A slurry reactor or a trickle bed reactor is particularly convenient. A trickle bed reactor is more particularly suitable. A trickle bed reactor fed at co-current by hydrogen and the glycerol is very particularly convenient. One or more reactors could be used.

In a simple way of carrying out the process according to the first embodiment of the invention, the process can be carried out discontinuously, in the following manner. An autoclave which is provided with a stirring or mixing unit and which can be thermostated is charged, in a suitable manner, with glycerol to be hydrogenated, the catalyst and a possible solvent. Thereafter, hydrogen is forced in until the desired pressure is reached, and the mixture is heated to the chosen reaction temperature while mixing thoroughly. The course of the reaction can be readily monitored by measuring the amount of hydrogen consumed, which is compensated by feeding in further hydrogen until the targeted conversion for glycerol is reached.

The mixture present after the hydrogenation can be worked up, for example, as follows : when the hydrogenation is complete, the reaction vessel is cooled, the pressure is let down and the catalyst is filtered off and rinsed with the possible solvent used, and the possible solvent used is then removed under reduced pressure or under atmospheric pressure. The crude product which remains can likewise be purified further by distillation under reduced pressure or under atmospheric pressure. When high-boiling solvents are used, it is also possible first to distil off the propanediol. When no solvent is used the mixture can be submitted directly to a distillation under reduced pressure or under atmospheric pressure. The recovered catalyst e.g by filtration can be reused in the discontinuous process as such or after reactivation by a physico-chemical treatment.

In another way of carrying out the process according to the first embodiment of the invention, the process can be carried out continuously, in the following manner: a vertical cylindrical reactor which can be thermostated is charged with the catalyst provided in a suitable shape in order to obtain a fixed bed of catalyst in the reactor. The reactor is fitted on its top part with inlet ports to feed the glycerol to be hydrogenated, neat or dissolved in a solvent and hydrogen, on its bottom part with an outlet port to recover the reaction mixture, with a regulation pressure device, and a vessel, to recover the reaction mixture and separate the liquid phase and the gas phase. Thereafter, hydrogen is introduced in the reactor until the desired pressure is reached, then the reactor is heated to the chosen reaction temperature, hydrogen and the glycerol, neat or dissolved in a solvent, are forced continuously in the reactor at the selected molar ratio and the reaction mixture is collected continuously in the recovery vessel. The extent of the reaction can be readily monitored by analyzing the composition of the liquid separated in the collection vessel. The liquid mixture present in the recovery vessel can be worked up, for example, by distillation under reduced pressure or under atmospheric pressure.

When the process according to the first embodiment of the invention is carried out continuously, a continuous process purge of gaseous by-products of the reaction or of gaseous contaminants of raw materials may be present and the main part of the non-reacted hydrogen can be recycled to the reactor.

Gas chromatography is usually used for assessing the content of the organic compounds in samples withdrawn at various stages of the process.

The present invention also relates in a second embodiment to a process for making a polymer selected from the group consisting of a polyether, a polyurethane, a polyester, and any mixture thereof, comprising obtaining 1,3-propanediol from the process according to the first embodiment, and further using said 1,3-propanediol as raw materials.

The process for making the polyether comprises obtaining 1,3-propanediol according to the first embodiment, and further submitting said 1,3-propanediol to a reaction with at least one compound selected from the group consisting of a halogenated organic compound, an organic epoxide, an alcohol, or any mixture thereof.

The process for making the polyurethane comprises obtaining 1,3-propanediol according to the first embodiment, and further submitting said 1,3-propanediol to a reaction with a polyisocyanate, preferably a diisocyanate.

In the process for making the polymer according to the invention, the polymer is preferably a polyester.

The present invention therefore also relates to a process for making a polyester comprising obtaining 1,3-propanediol according to the first embodiment and further submitting said 1,3-propanediol to a reaction with a carboxylic acid and/or a carboxylic acid ester.

The features mentioned above for the process for manufacturing the 1,3-propanediol are applicable for obtaining the 1,3-propanediol used for making the polymer, preferably a polyester.

In the process for making a polyester according to the invention, the carboxylic acid is preferably a polycarboxylic acid, more preferably a dicarboxylic acid. The polycarboxylic acid is preferably selected from the group consisting of an aliphatic acid, saturated or unsaturated, an aromatic acid, an alkylaromatic acid, saturated or unsaturated, a heteroaromatic acid, an alkylheteroaromatic acid, saturated or unsaturated, or any mixture thereof.

The preferred aliphatic dicarboxylic acid contains from 2 to 16 carbon atoms and is more preferably selected from the group consisting of oxalic acid, malonic acid, succinic acid, glutaric acid, adipic acid, sebacic acid, azelaic acid and any mixture thereof.

The preferred unsaturated dicarboxylic acid is preferably selected from the group consisting of fumaric acid, maleic acid, and any mixture thereof.

The preferred aromatic dicarboxylic acid is preferably selected from the group consisting of o-phthalic acid, m-phthalic acid, p-phthalic acid (terephthalic acid), naphthalene dicarboxylic acid, and any mixture thereof. The preferred aromatic dicarboxylic acid is more preferably terephthalic acid.

The preferred alkyl aromatic dicarboxylic acid is preferably selected from the group consisting of 4-methylphthalic acid, 4-methylphthalic acid, and any mixture thereof.

The preferred unsaturated dicarboxylic aromatic acid is preferably selected from the group consisting of vinyl phthalic acids, and any mixture thereof.

The preferred heteroaromatic dicarboxylic acid is preferably selected from the group consisting of furano dicarboxylic acids, and any mixture thereof, and is preferably 2,5-furano dicarboxylic acid.

In the process for making a polyester according to the invention, the carboxylic acid ester is preferably an ester of the above cited dicarboxylic acid, preferably a methyl, or ethyl ester. The preferred ester is selected from the group consisting of an ester of terephthalic acid, an ester of furano dicarboxylic acid, and any mixture thereof. The ester is more preferably a terephthalic acid ester, and most preferably dimethyl terephthalate.

The production of the polyester is described, for example, in Ullmann's Encyclopedia of Industrial Chemistry, Horst Köpnick, Manfred Schmidt, Wilhelm Brügging, Jörn Rüter and Walter Kaminsky, Published Online : 15 JUNE 2000, DOI : 10.1002/14356007.a21_227, pages 623-649.

The polymer, preferably a polyester, obtained according to the process of the invention usually exhibits a ¹⁴C/¹²C higher than or equal to 0.33 10⁻¹², often higher than or equal to 0.5 10⁻¹², frequently higher than or equal to 0.75 10⁻¹², in many case higher than or equal to 1.0 10⁻¹² and in particular higher than or equal to 1.1 10⁻¹².

In a further embodiment, the invention also relates to a polyester exhibiting a ¹⁴C/¹²C higher than or equal to 0.33 10⁻¹², often higher than or equal to 0.5 10⁻¹², frequently higher than or equal to 0.75 10⁻¹², in many case higher than or equal to 1.0 10⁻¹² and in particular higher than or equal to 1.1 10⁻¹².

The polyester is preferably obtainable by reacting 1,3-propanediol obtained from the process according to the first embodiment, and further submitting said 1,3-propanediol to a reaction with a carboxylic acid and/or a carboxylic acid ester, as described here above.

The polyester is more preferably obtained by reacting 1,3-propanediol obtained from the process according to the first embodiment, with a carboxylic acid and/or a carboxylic acid ester, as described here above.

The present invention also relates in a third embodiment to a process for making a polyester fiber comprising obtaining 1,3-propanediol from the process according to the first embodiment, submitting said 1,3-propanediol to a reaction with a carboxylic acid and/or a carboxylic acid ester to obtain a polyester according to the second embodiment and further converting said polyester into a fiber.

The methods for measuring the ¹⁴C content are precisely described in standards ASTM D 6866 (notably D 6866-06, D 6866-08 and D 6866-12) and in standards ASTM 7026 (notably D 7026-04). The method preferably used is described in standard ASTM D6866-12.

The features mentioned above for the process for manufacturing the 1,3-propanediol and for the process for making the polyester are applicable for obtaining the 1,3-propanediol and the polyester used for making the polyester fiber.

The production of the polyester fibers is described, for example, in Ullmann's Encyclopedia of Industrial Chemistry, Helmut Sattler and Michael Schweizer, Published Online : 15 OCT 2011, DOI : 10.1002/14356007.o10_o01, pages 1 to 34.

The polyester fibers have numerous applications and can be used in tires, rope, cordage, sewing thread, seat belts, hoses, webbing, coated fabrics, carpets, apparel, home fashions, upholstery, medical, interlinings, filtration, fiberfill, high-loft, roofing, geotextiles, and substrates, for instance.

In a fourth embodiment, the invention relates to 1,3-propanediol obtainable according to the process of the first embodiment.

In a fifth embodiment, the invention also relates to the use of the 1,3-propanediol of the fourth embodiment in the manufacture of a polyester.

The features mentioned above for the process for manufacturing the polyester according to the second embodiment are applicable to the use of the fifth embodiment.

In a sixth embodiment, the invention also relates to a polyester obtainable according to the process of the fifth embodiment.

The features mentioned above for the polyester obtained according to the second embodiment are applicable to the polyester of the sixth embodiment.

In a seventh embodiment, the invention relates to the use of the polyester of the sixth embodiment in the manufacture of a polyester fiber.

In an eight embodiment, the invention also relates to a process for preparing a supported catalyst comprising at least one first element selected from iridium, rhodium, palladium, platinum and combination thereof, at least one second element selected from chromium, molybdenum, tungsten and any combination thereof, and rhenium, said compounds and rhenium being supported on alumina comprising:
a) Providing an alumina support;
b) Impregnating said support with an aqueous solution containing at least one precursor of the second element and at least one precursor of rhenium;
c) Removing the water of b) in order to obtain a first dried solid;
d) Heating the first dried solid of c) with a gas containing oxygen in order to obtain a first calcined solid;
e) Optionally repeating b) to d) where the support is the calcined solid of d);
f) Impregnating the first calcined solid of d) or e) with an aqueous solution of at least one precursor of the first element;
g) Removing the water of step f) in order to obtain a second dried solid;
h) Heating the second dried solid of g) with a gas containing oxygen in order to obtain a second calcined solid;
i) Treating the second calcined solid of h) with a gas containing hydrogen in order to obtain the supported catalyst.

The impregnation steps (b) and (f) can be carried out according to any known technique of impregnation as described in "Applied Catalysis A: General, 1995, 133,281-292". Successive wet-impregnation is preferred. Any solvent can be used provided that the precursors of the first element, of the element and of rhenium are soluble therein. Water is a preferred solvent. Any precursors of the first element, of the second element and of rhenium can be used provided that they are soluble in the solvent used. When the first element is platinum, the second element is tungsten, chloroplatinic acid, ammonium metatungstate and ammonium perrhenate are preferred, in particular when the solvent used is water.

The drying of steps (c) and (g) can be carried out according to any known technique. Drying is carried out at a temperature preferably higher than or equal to 50°C, more preferably higher than or equal to 70°C, and most preferably higher than or equal to 90°C. This drying temperature is preferably lower than or equal to 180°C, more preferably lower than or equal to 150°C, and most preferably lower than or equal to 130°C. Drying is carried out at a pressure preferably higher than or equal to 400 mbar absolute, more preferably higher than or equal to 600 mbara, and most preferably higher than or equal to 800 mbara. This drying pressure is preferably lower than or equal to 1800 mbara, more preferably lower than or equal to 1500 mbara, and most preferably lower than or equal to 1200 mbara.

The calcination of steps (d) and (h) can be carried out according to any known technique. Calcination is carried out at a temperature preferably higher than or equal to 300°C, more preferably higher than or equal to 350°C, and most preferably higher than or equal to 400°C. This calcination temperature is preferably lower than or equal to 700°C, more preferably lower than or equal to 650°C, and most preferably lower than or equal to 600°C. The calcination is usually carried out with a gas containing oxygen, preferably with air.

The treatment under hydrogen of step (i) can be carried out at a temperature preferably higher than or equal to 150°C, more preferably higher than or equal to 160°C, and most preferably higher than or equal to 170°C. This treatment temperature is preferably lower than or equal to 400°C, more preferably lower than or equal to 350°C, and most preferably lower than or equal to 325°C.

All the features disclosed here above for the first element, the second element, rhenium, alumina and hydrogen, are applicable to the process for preparing the catalyst.

The examples below are intended to illustrate the invention without, however, limiting it.

### Examples 1 to 5

### 1. Catalyst preparation

### Catalysts 1 to 5

The supported catalysts 2 to 4 were prepared by the successive wet-impregnating method. The support was impregnated with an aqueous solution (5 g H₂O/g support) of ammonium metatungstate hydrate (Aldrich) and ammonium perrhenate (Aldrich) at about 25 °C for about 60 minutes, under agitation in a rotary evaporator. The water was removed by using rotary evaporator at 50 °C, at about 23 mbara (vacuum with a water jet pump at 20°C) and for about 30 minutes, and the resulting sample was dried overnight at 110 °C under static air under 1 bara. The dried sample was then calcined at 450 °C under static air, at 1 bara, for 3 hours. The calcined sample was slurried with an aqueous solution of chloroplatinic acid hydrate (Aldrich, ∼ 38% Pt basis) (5 g H₂O/g calcined sample). The water was removed as above, the sample containing platinum was dried at 110 °C as above, calcined at 450 °C as above, and treated at 300 °C with flowing hydrogen (2.2-2.9 mlN/min/g of catalyst) for 3 hours. The following alumina based oxide has been used as: gamma-Al₂O₃ (Puralox TH 100/150) was provided by Sasol as powder and used as received. The catalysts have been obtained as powders.

The same procedure has been followed for catalyst 1 except that no rhenium compound was used.
The weight ratios of Pt and WO₃ in the final Pt/WO₃₋ReOx/Al₂O₃ catalysts are respectively of 5 wt% (Pt) and 10 wt% (WO₃). The weight ratio is indicated in Table 1 here below.

**Table 1**

| Catalyst n° | Pt (wt%) | WO₃ (wt%) | Re (wt%) | Re (wt%)-determined by ICP | S_{BET} of support (m²/g) | Vₚ of support (cm³/g) | Alumina based support |
|---|---|---|---|---|---|---|---|
| 1 | 5 | 10 | 0 | | 150 | 1.1 | Puralox TH 100/150 |
| 2 | 5 | 10 | 0.5 | 0.35 | ibid | ibid | ibid |
| 3 | 5 | 10 | 1 | 0.84 | ibid | ibid | ibid |
| 4 | 5 | 10 | 2 | 1.7 | ibid | ibid | ibid |
| 5 | 5 | 10 | 4 | 3.3 | ibid | ibid | ibid |

### 2. Catalyst evaluation

The catalysts have been used as obtained after reduction.

For examples 1 to 5, the hydrogenation of glycerol has been carried out in a 100 ml Hastelloy (C-22) autoclave.

2.25 g catalyst and 50 g of a mixture of glycerol (VWR, vegetal origin, 99.5 % containing 0.73 g/kg of water) and water (Milli-Q water) and it was then placed in the autoclave. Subsequently, The autoclave was sealed and purged with nitrogen (N₂, Air Product, 99.998%) by pressurizing and depressurizing several times and then with hydrogen (H₂, Air Product, 99.9995%) by pressurizing and depressurizing several times, and then heated to the desired temperature of 160 °C. Meanwhile, the hydrogen pressure was increased to 60 bara and mechanical stirring was set at 1000 rpm. The time zero for the reaction was defined as the time at which the stirring was started. During the reaction, the pressure was always maintained at 60 bara. After an appropriate reaction time, the reaction was stopped by cooling down the autoclave. The pressure was released. The liquid product was separated by using polypropylene filter (0.8 µm of porosity) and then analyzed by gas chromatography (GC).

### 3. Analyses

The GC analyses of the liquids have been carried out under the following conditions. The sample has been dissolved in dimethylformamide. The GC has been has been performed by injecting the solution on a CP Wax 57 CB column (25m*0.25mm*0.2um) using an appropriate temperature program and flame ionisation detection. Quantification has been done using an internal standard (2-chlorotoluene) and relative response factors, determined using standard reference products. The specific GC conditions were as follows:
- Injector temperature: 250 °C
- Detector temperature: 280 °C
- Oven temperature: 60 °C (5 min) - 10 °C/min - 240 °C (5 min)
- Split flow: 60 ml/min
- Flow rate: 1 ml/min (constant)

The mass balance was calculated for each test and it was found higher than 95 % in most cases.

The conditions for carrying out examples 1 to 5 are summarized in Table 2.

**Table 2**

| Example | Catalyst | Reaction | | | | |
|---|---|---|---|---|---|---|
| | N° | water (g) | glycerol (g) | T (°C) | P (bara) | Time (h) |
| 1 | 1 | 20 | 30 | 160 | 60 | 15 |
| 2 | 2 | 20 | 30 | 160 | 60 | 14 |
| 3 | 3 | 20 | 30 | 160 | 60 | 13.5 |
| 4 | 4 | 20 | 30 | 160 | 60 | 14.5 |
| 5 | 5 | 20 | 30 | 160 | 60 | 14.5 |

The results of the tests are summarized in Table 3 here below.

The selectivity of the various products has been calculated as follows:
Selectivity product = 100 X [(number of mole of product recovered in the liquid reaction mixture at the end of reaction)/(sum of number of mole of 1,3-propanediol, 1,2-propanediol, 1-propanol and 2-propanol recovered in the liquid reaction mixture at the end of reaction)].

The TOF value in Table 3 is the ratio between the number of kilogram of converted glycerol and the number of kilogram of platinum in the catalyst used, *i.e.* utilized, per hour.

**Table 3**

| Example | Product selectivity (% mol) | | | | TOF |
|---|---|---|---|---|---|
| | 1,3-propanediol | 1,2-propanediol | 1-propanol | 2-propanol | |
| 1 | 55.5 | 5.1 | 30 | 9.4 | 5.17 |
| 2 | 53.6 | 5.2 | 32.1 | 9 | 6.53 |
| 3 | 54.6 | 4.7 | 30.8 | 9.8 | 7.24 |
| 4 | 52.8 | 5.4 | 31.4 | 10.4 | 6.75 |
| 5 | 48.7 | 6.5 | 32.9 | 11.8 | 7.83 |

## Claims

1. A process for manufacturing 1,3-propanediol by reacting glycerol with hydrogen in the presence of a supported catalyst, the supported catalyst comprising at least one first element selected from iridium, rhodium, palladium, platinum and any combination thereof, at least one second element selected from chromium, molybdenum, tungsten and any combination thereof, and rhenium, said elements and rhenium being supported on alumina.

2. The process according to claim 1, wherein the alumina in the supported catalyst is selected from gamma alumina, delta alumina, theta alumina, and any mixture thereof.

3. The process according to claim 1 or 2, wherein in the supported catalyst at least part of the first element is present under metallic form, at least part of the second element is present under oxide form and at least part of rhenium is present under oxide form.

4. The process according to any one of claims 1 to 3, wherein in the supported catalyst the first element is platinum and the second element is tungsten.

5. The process according to any one of claims 1 to 4, wherein the content of rhenium with respect to the supported catalyst and expressed in weight percent of metal per weight of catalyst higher than or equal to 0.1 percent by weight and lower than or equal to 5 % by weight.

6. The process according to any one of claims 1 to 5, wherein the supported catalyst exhibits at least one of the following features:
• a nitrogen BET specific area higher than or equal to 50 m²/g and lower than or equal to 400 m²/g;
• a nitrogen BET total pore volume higher than or equal to 0.2 cm³/g and lower than or equal to 1.5 cm³/g;
• a nitrogen BET average pore size higher than or equal to 2 nm and lower than or equal to 150 nm;
• a TEM average particle size of the first compound lower than or equal to 15 nm;
• a content of the first element with respect to the supported catalyst and expressed in weight of metal per weight of catalyst higher than or equal to 0.1 percent by weight and lower than or equal to 10 % by weight; and
• a content of the second element with respect to the supported catalyst and expressed in weight of trioxide per weight of catalyst higher than or equal to 1 percent by weight and lower than 20 percent by weight.

7. The process according to any one of claims 1 to 6, carried out in the continuous mode and wherein the reaction is carried out in at least one of the following conditions:
• A temperature higher than or equal to 70 °C and lower than or equal to 300°C;
• A hydrogen partial pressure higher than or equal to 1 bar absolute and lower than or equal to 200 bar absolute;
• In a liquid reaction medium at a residence time of the liquid reaction medium higher than or equal to 5 min and lower than or equal to 25 h.

8. The process according to claim 7 carried out in a trickle-bed reactor.

9. A process for making a polyester comprising obtaining 1,3-propanediol according to the process of any one of claims 1 to 8, and further submitting said 1,3-propanediol to a reaction with a carboxylic acid and/or a carboxylic acid ester.

10. A process for making a polyester fiber comprising obtaining a polyester according to the process of claim 9, and further converting said polyester into a fiber.

11. 1,3-propanediol obtainable by the process of any one of claims 1 to 8.

12. Use of 1,3-propanediol according to claim 11 in the manufacture of a polyester.

13. A polyester obtainable by the process of claim 12.

14. Use of the polyester according to claim 13 in the manufacture of a polyester fiber.

15. A process for preparing a supported catalyst comprising at least one first element selected from iridium, rhodium, palladium, platinum and any combination thereof, at least one second element selected from chromium, molybdenum, tungsten and any combination thereof, and rhenium, said elements and rhenium being supported on alumina comprising:
a) Providing an alumina support;
b) Contacting said support with an aqueous solution containing at least one precursor of the second element and at least one precursor of rhenium;
c) Removing the water of b) in order to obtain a first dried solid;
d) Treating the first dried solid of c) with a gas containing oxygen in order to obtain a first calcined solid;
e) Optionally repeating b) to d) where the support is the calcined solid of d);
f) Contacting the first calcined solid of d) or e) with an aqueous solution of at least one precursor of the first element;
g) Removing the water of f) in order to obtain a second dried solid;
h) Treating the second dried solid of g) with a gas containing oxygen in order to obtain a second calcined solid;
i) Contacting the second calcined solid of h) with a gas containing hydrogen in order to obtain the supported catalyst.
